# EUROPEAN PATENT APPLICATION

(11) **EP 4 052 812 A1**
(43) Date of publication of application: **07.09.2022**
(21) Application number: 22157086.4
(22) Date of filing: 16.02.2022
(51) Int. Cl.: B08B 3/02, A47F 10/04, A61L 2/22, A61L 2/24, B08B 9/08, B08B 9/093, B08B 9/20, B08B 9/28, B08B 9/34, B08B 9/42, B60S 3/04, B60S 9/04

(54) **APPARATUS FOR SANITIZING SHOPPING CARTS**

(30) Priority: 02.03.2021 IT 202100004787
(71) Applicant: Barbieri, Claudio, 42048 Rubiera (RE) (IT)
(72) Inventor: Barbieri, Claudio, 42048 Rubiera (RE) (IT)
(74) Representative: Modiano, Micaela Nadia

(57) **Abstract**

An apparatus (1) for sanitizing shopping carts, comprising a framework (2) adapted to form a passage tunnel for a cart (3) to be sanitized, the framework (2) being provided with nozzles (6) for sanitizing a cart (3) that passes within the framework (2), and retention means (10)adapted to allow the locking of at least one wheel of the cart that has entered the framework (2), for its correct positioning below the nozzles (6), for complete sanitizing of the cart.

## Description

The present invention relates to an apparatus for sanitizing shopping carts. More particularly, the invention relates to an apparatus for sanitizing shopping carts which allows to sanitize any type of cart as regards dimensions and shapes.

As is known, apparatuses for the sanitizing of shopping carts are available on the market. These apparatuses consist of a sort of tunnel through which the shopping cart to be sanitized must pass. Appropriate nozzles sanitize the cart once it has entered the tunnel, and then make it exit at the end of the sanitizing procedure.

Sanitizing solutions of the known type therefore provide for apparatuses designed for certain type of carts as regards dimensions and shapes.

In fact, the position of the cart within the apparatus depends on its size and therefore the positioning of the nozzles for sanitizing, which occurs mainly at the cart handle, must be provided for a specific size of said cart.

This means that if carts with different dimensions were to be used, the positioning of the cart sanitizing nozzles would not be optimum as regards particularly cart handle sanitizing. The nozzles may in fact direct the sanitizing jet to a position that does not correspond to the position in which the cart handle is located when the cart is inserted in the sanitizing apparatus.

The aim of the present invention is to provide an apparatus for sanitizing shopping carts that allows to sanitize any type of cart in terms of shapes and dimensions.

Within this aim, an object of the present invention is to provide an apparatus for sanitizing shopping carts that allows to ensure a substantially total removal of the viral burden for any type of cart that might be provided.

Another object of the present invention is to provide an apparatus for sanitizing shopping carts that allows to position the cart automatically in a correct position for its sanitizing.

Not least object of the present invention is to provide an apparatus for sanitizing shopping carts that is highly reliable, relatively easy to provide and at competitive costs.

This aim and these and other objects which will become better apparent hereinafter are achieved by an apparatus for sanitizing shopping carts, comprising a framework adapted to form a passage tunnel for a cart to be sanitized, said framework being provided with nozzles for sanitizing a cart that passes within said framework, characterized in that it comprises retention means adapted to allow the locking of at least one wheel of the cart that has entered said framework, for its correct positioning below said nozzles, for complete sanitizing of said cart.

Further characteristics and advantages of the invention will become better apparent from the description of preferred but not exclusive embodiments of the apparatus according to the invention, illustrated by way of non-limiting example in the accompanying drawings, wherein:

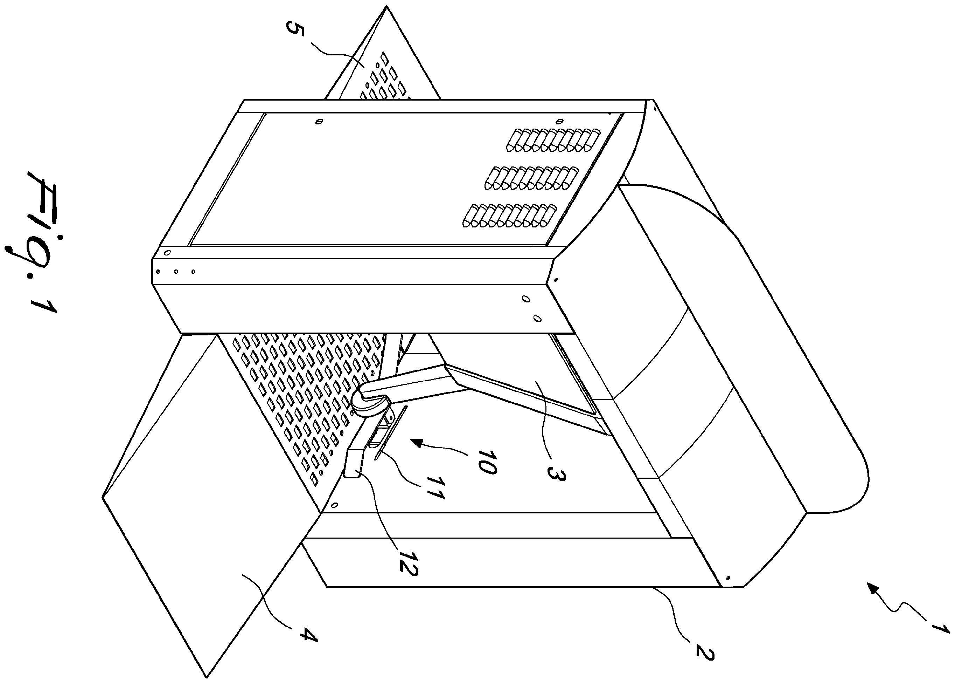

Figure 1 is a perspective view of the apparatus according to the present invention, with a cart in a first position;

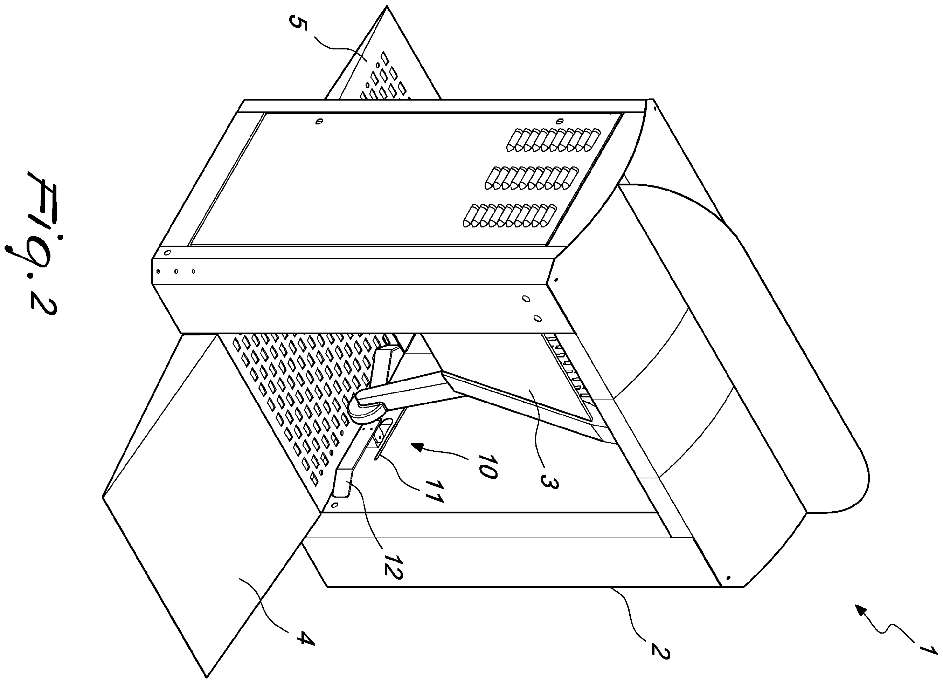

Figure 2 is a perspective view of the apparatus according to the present invention, with the cart in a second position;

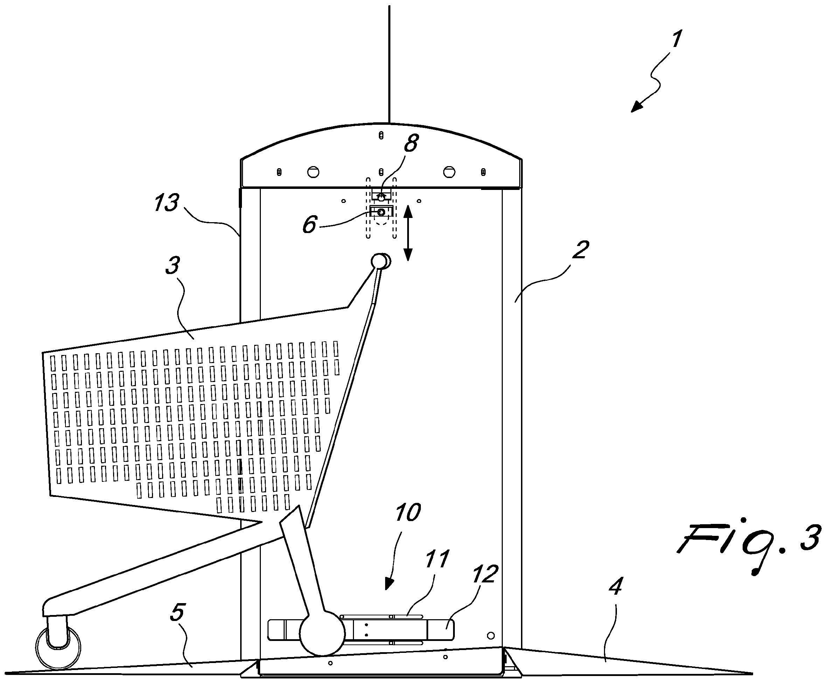

Figure 3 is a lateral elevation view of the apparatus according to the present invention;

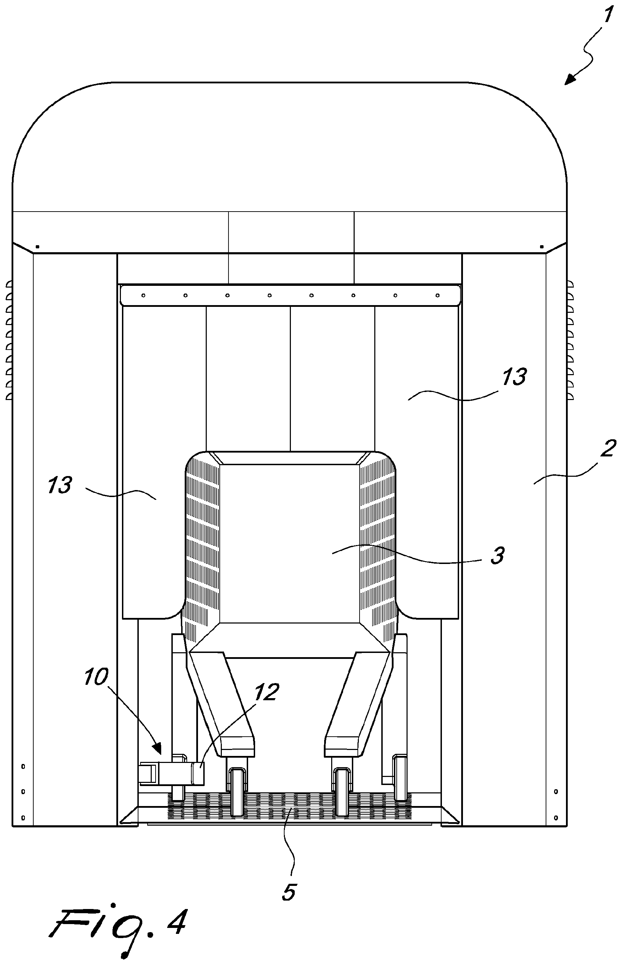

Figure 4 is a front elevation view of the apparatus according to the present invention;

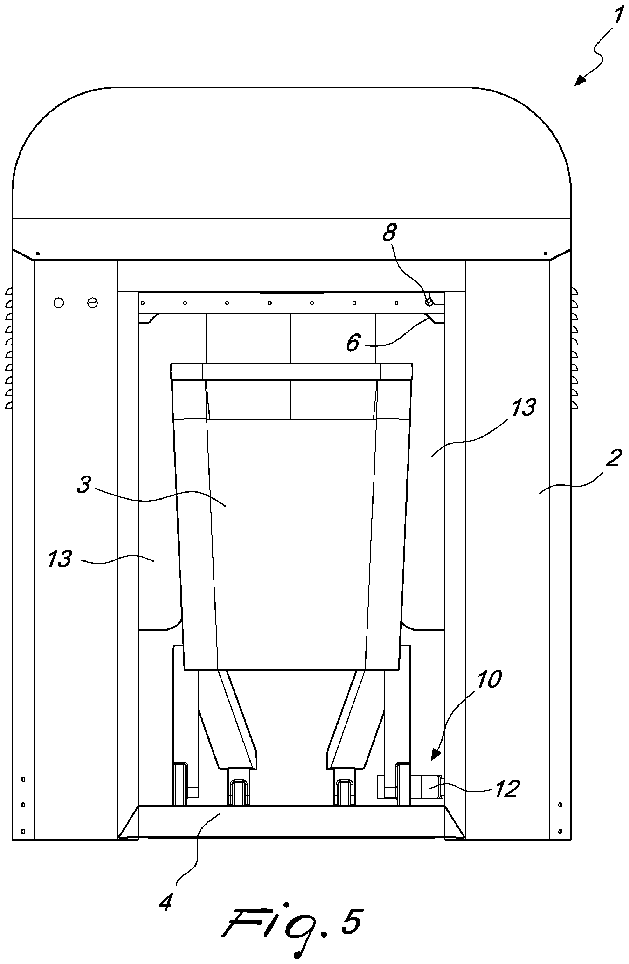

Figure 5 is a rear elevation view of the apparatus according to the present invention.

With reference to the figures, the sanitizing apparatus according to the invention, generally designated by the reference numeral 1, comprises a framework 2 adapted to form a tunnel through which a shopping cart 3 is adapted to pass.

The apparatus is provided, at the inlet region of the cart 3, with an entry ramp 4 which allows the cart to access the framework 2, said entry ramp 4 blending with a descent ramp 5 which starts substantially already at the inlet region of the framework 2.

The descent ramp 5 allows the cart to be arranged in the desired position.

Sanitizing nozzles 6 are conveniently provided at the upper region of the framework 2 and are arranged at the opposite sides of the framework 2.

Preferably, the nozzles 6 are arranged at both sides of the framework, but they can also be provided at a single side, or the nozzles 6 can be provided in a central position with respect to the framework 2.

In any case, the nozzles 6 can move along vertical guides 8 (and preferably also along horizontal guides), so as to arrange the nozzles 6 in the optimum position with respect to the longitudinal extension of the framework 2 and in the optimum position with respect to the height of the cart 3 that is made to pass within the framework 2.

Conveniently, the cart 3 can be locked within the framework 2, once the cart has been made to descend along the descent ramp 5, so as to be able to arrange it in the correct position, so that the nozzles 6 can sanitize the handle, the child seat and the basket.

Conveniently, the nozzles 6 must be capable of sanitizing mainly the handle of the cart 3, which is the part of the cart that is most subject to contaminations, since the user rests his hands on it in order to push it.

In order to lock the cart 3 within the framework 2 in the correct position, retention means 10 are provided at the sides of the framework 2 and allow to lock a wheel of the cart and keep it centered at the desired position.

Conveniently, the retention means 10 can be provided bilaterally, therefore at the two opposite side walls of the framework 2, or it is possible to provide a single retention means 10.

Advantageously, the retention means 10 each comprise a lateral slider 11 along which the actual retainer 12, which is an element shaped so as to contain the movement of the rear wheel of the cart, can be made to slide, therefore locking the cart in position under the nozzles 6.

The apparatus according to the invention substantially allows to make a cart 3 pass within the framework 2, first making it climb the ramp 4 and then allowing it to descend along the ramp 5 until the cart 3 is locked, with its rear wheel or preferably with both rear wheels, at the retention means 10 arranged laterally at the base of the opposite side walls of the framework 2.

In this position, one is certain that the cart 3 is located exactly at the nozzles 6 so that it can be sanitized.

In any case, the nozzles 6, as mentioned, can move both longitudinally along the longitudinal extension of the framework 2 and vertically (or only vertically or only longitudinally) so as to move closer to or away from the cart 3, so as to adapt to any type of cart.

In order to appropriately center the cart 3 within the framework 2, it is possible to provide centering means 13 which keep the cart in the exact center of the framework and adapt to different widths of the cart.

Furthermore, the retention means 10 can be provided, instead of at the side walls of the framework 2, at the bottom region of the framework 2.

In practice it has been found that the sanitizing apparatus according to the invention fully achieves the intended aim and objects, since it allows to sanitize carts of different sizes.

The apparatus thus conceived is susceptible of numerous modifications and variations, all of which are within the scope of the accompanying claims.

Thus, for example, it is possible to provide a vision system arranged at the inlet side of the framework 2, which is capable of detecting the size of the cart, and therefore to allow an automatic positioning both of the retention means 10 and of the nozzles 6, according therefore to the type of cart, thus automating the entire cart sanitizing treatment without the need for the operator, depending on the cart, to position correctly the retention means 10 and the nozzles 6.

This can be useful if for example a supermarket has carts of different sizes available at the same time, which must however be all sanitized with the same sanitizing apparatus according to the invention.

All the details may furthermore be replaced with other technically equivalent elements.

In practice, the materials used, as well as the contingent shapes and dimensions, may be any according to the requirements and the state of the art.

The disclosures in Italian Patent Application No. 102021000004787 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An apparatus (1) for sanitizing shopping carts (3), comprising a framework (2) adapted to form a passage tunnel for a cart (3) to be sanitized, said framework (2) being provided with nozzles (6) for sanitizing a cart (3) that passes within said framework (2), **characterized in that** it comprises retention means (10) adapted to allow the locking of at least one wheel of the cart (3) that has entered said framework (2), for its correct positioning below said nozzles (6), for complete sanitizing of said cart (3).

2. The apparatus according to claim 1, **characterized in that** said nozzles (6) are adjustable in position at least longitudinally or vertically.

3. The apparatus according to claim 1, **characterized in that** said retention means (10) comprise at least one retention means (12) arranged at one of the opposite side walls of said framework (2), proximate to the lower region of the wall, so as to lock at least one rear wheel of said cart (3).

4. The apparatus according to one or more of the preceding claims, **characterized in that** said retention means (10) comprise a guide (11) adapted to allow the positioning of said retention means (12) at the desired position in order to lock the rear wheel of the cart (3).

5. The apparatus according to one or more of the preceding claims, **characterized in that** it comprises retention means (10) arranged at both mutually opposite side walls of said framework (2) in order to lock the two rear wheels of said cart (3).

6. The apparatus according to one or more of the preceding claims, **characterized in that** said retention means (10) are arranged at the bottom region of said framework (2).

7. The apparatus according to one or more of the preceding claims, **characterized in that** said nozzles (6) can move both along a horizontal guide arranged along the longitudinal extension of said framework (2) and along a vertical guide (7) so as to move closer to or away from said cart (3).

8. The apparatus according to one or more of the preceding claims, **characterized in that** it comprises centering means (13) adapted to allow to center said carriage within said framework and to adapt to different widths of said cart (3).

9. The apparatus according to one or more of the preceding claims, **characterized in that** it comprises an entry ramp (4) and a descent ramp (5) for the passage of the cart into and out of the framework (2).

10. The apparatus according to one or more of the preceding claims, **characterized in that** it comprises a vision system adapted to detect the size of said cart (3) and to allow the automatic positioning of said retention means (10) and of said nozzles (6), depending on the detected size, in order to allow complete and quick sanitizing of said cart (3).
